# EUROPEAN PATENT APPLICATION

(11) **EP 4 509 057 A1**
(43) Date of publication of application: **19.02.2025**
(21) Application number: 23191863.2
(22) Date of filing: 17.08.2023
(51) Int. Cl.: A61B 6/04, G06T 7/00

(54) **METHOD AND SYSTEM FOR VISUALIZING CHANGE IN POSITION**

(71) Applicant: Koninklijke Philips N.V., 5656 AG Eindhoven (NL)
(72) Inventor: LUNDT, Bernd, 5656 AG Eindhoven (NL); KOEPNICK, Johannes, 5656 AG Eindhoven (NL); MAY, Jan Marek, 5656 AG Eindhoven (NL)
(74) Representative: Philips Intellectual Property & Standards

(57) **Abstract**

A system and method for visualizing a change in position of an individual during a medical imaging procedure for the individual. A reference image, in which the individual has a desired position for the medical imaging procedure, is obtained and processed to produce a reference contour bounding a region of interest. A later image (e.g. a current image) of the individual is obtained, and a display is controlled to provide a visual representation of the shape of the region of interest in the later image and, at least when one or more predetermined conditions are met, the reference contour.

## Description

### FIELD OF THE INVENTION

The invention relates to the field of medical imaging.

### BACKGROUND OF THE INVENTION

During a medical imaging procedure, it is important that an area of the body being imaged is correctly positioned with respect to the medical imaging apparatus in order to obtain an image with a desired anatomical view and of sufficient image quality.

Typically, an individual to be imaged is guided or moved into a correct position by an imaging operator (e.g. a radiographer). However, there is frequently a delay between achieving the correct position and acquiring the medical image, for example, in order for the imaging operator to move to a position in which they are not exposed to X-ray radiation, in the case of an X-ray imaging procedure. During this delay, the individual being imaged may move, which may result in a deviation from the correct position that results in acquiring an incorrect view of the area of the body being imaged and/or an image of insufficient quality. If this occurs, the medical imaging procedure may need to be repeated, requiring additional clinical time and, in the case of imaging modalities that use radiation, increasing the individual's radiation dose.

In order to reduce a likelihood of acquiring images that are not clinically useful, image operators require information that allows them to detect, immediately before triggering image acquisition, whether an unacceptable movement of the individual has occurred.

### SUMMARY OF THE INVENTION

The invention is defined by the claims.

According to examples in accordance with an aspect of the invention, there is provided a computer-implemented method for visualizing a change in position of an individual during a medical imaging procedure for the individual, the computer-implemented method comprising: obtaining a reference image of an individual captured at a first point in time using a non-penetrative imaging device; performing contour detection on the reference image to produce a reference contour that bounds a representation of a region of interest of the individual within the reference image; obtaining a further image of the individual captured at a second point in time using the non-penetrative imaging device; and controlling a display to provide a visual representation of: at least the shape of a representation of the region of interest within the further image; and at least when movement of the region of interest between the first point in time and the second point in time meets one or more predetermined conditions , the reference contour.

By displaying at least the shape of a representation of the region of interest within the further image (e.g. by displaying the further image and/or a contour of the representation of the region of interest) and the reference contour, an image operator may detect a difference in the position of the region of interest between the reference image and further image, and may assess whether repositioning of the individual is required before acquisition of a medical image.

For instance, the reference contour may, at least when the movement of the region of interest meets the one or more predetermined conditions, be superimposed on the visual representation of the shape of the representation of the region of interest in the further image.

The further image of the individual is preferably a live image of the individual. The steps of obtaining a further image of the individual and controlling the display may be continuously repeated in real time.

In some examples, the step of controlling the display comprises controlling the display to provide a visual representation of the reference contour only when the movement of the region of interest between the first point in time and the second point in time meets the one or more predetermined conditions.

In this way, clinical time is not wasted assessing the position of the individual in situations where no movement, or no relevant movement, has occurred.

In some examples, the one or more predetermined conditions comprises a first condition that a measure of movement exceeds a first predetermined threshold.

In some examples, the computer-implemented method further comprises: performing contour detection on the further image to produce a further contour that bounds a representation of the region of interest within the further image; and processing the reference contour and the further contour to determine the measure of movement.

In some examples, the measure of movement is a displacement of the region of interest between the first point in time and the second point in time.

In some examples, one or more properties of the visual representation of the reference contour, when displayed, changes responsive to the measure of movement.

For instance, the color of the visual representation of the reference contour may change responsive to the measure of movement, e.g. a first color may be used in response to the measure of movement exceeding a second predetermined threshold, and a second color may be used in response to the measure of movement failing to exceed the second predetermined threshold. In this way, an image operator may easily identify unacceptable levels of movement.

In some examples, the computer-implemented method further comprises performing contour detection on the further image to produce a further contour that bounds a representation of the region of interest within the further image; and the step of controlling the display to provide the visual representation of at least the shape of the representation of the region of interest within the further image comprises, at least when movement of the region of interest between the first point in time and the second point in time meets the one or more predetermined conditions, controlling the display to provide a visual representation of the further contour.

In some examples, the step of controlling the display comprises controlling the display to provide a visual representation of the further contour only when the movement of the region of interest between the first point in time and the second point in time meets the one or more predetermined conditions.

In some examples, one or more properties of the visual representation of the further contour, when displayed, change responsive to the measure of movement.

In some examples, the step of controlling the display to provide the visual representation of at least the shape of the representation of the region of interest within the further image consists of controlling the display to provide a visual representation of the further contour.

In other words, only contours are provided at the display (e.g. only the further contour, or the reference contour and the further contour). This maintains the privacy of the individual, in particular, where the display is visible to others in addition to the image operator conducting the medical imaging procedure.

In some examples, the step of controlling the display to provide the visual representation of at least the shape of the representation of the region of interest within the further image comprises controlling the display to provide a visual representation of the further image.

In some examples, the visual representation of the further image is an abstraction of the further image. This again maintains the privacy of the individual, by conveying position information for the individual without displaying identifying details of the individual. For instance, the visual representation of the further image may be a blurred (or otherwise obscured) version of the further image, a representation comprising a silhouette of the individual, or, depending on the type of image acquired by the non-penetrative imaging device, a depth visualization (i.e. an image displaying only depth information) or a thermogram.

In some examples, the reference image is a depth image.

In some examples, the step of performing contour detection comprises performing threshold-based contour detection.

There is also proposed a computer program product comprising computer program code means which, when executed on a computing device having a processing system, cause the processing system to perform the steps of any of the methods described above.

There is also proposed a computer-readable medium comprising instructions which, when executed by a computer, cause the computer to carry out the steps of any of the methods described above.

According to another aspect of the invention, there is provided a processing system for visualizing a change in position of an individual during a medical imaging procedure for the individual, the processing system being configured to: obtain a reference image of an individual captured at a first point in time using a non-penetrative imaging device; perform contour detection on the reference image to produce a reference contour that bounds a representation of a region of interest of the individual within the reference image; obtain a further image of the individual captured at a second point in time using the non-penetrative imaging device; and control a display to provide a visual representation of: at least the shape of a representation of the region of interest within the further image; and at least when movement of the region of interest between the first point in time and the second point in time meets one or more predetermined conditions, the reference contour.

There is also proposed an imaging system, comprising: a non-penetrative imaging device; and the processing system described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an imaging system, according to an embodiment of the invention;
Fig. 2 illustrates a first set of example visualizations according to a first embodiment;
Fig. 3 illustrates a second set of example visualizations according to a second embodiment;
Fig. 4 illustrates a third set of example visualizations according to a third embodiment;
Fig. 5 illustrates a fourth set of example visualizations according to a fourth embodiment;
Fig. 6 illustrates a fifth set of example visualizations according to a fifth embodiment;
Fig. 7 illustrates a sixth set of example visualizations according to a sixth embodiment;
Fig. 8 illustrates a seventh set of example visualizations according to a seventh embodiment;
Fig. 9 illustrates an eighth set of example visualizations according to an eighth embodiment; and
Fig. 10 illustrates a computer-implemented method for visualizing a change in position of an individual during a medical imaging procedure for the individual, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for visualizing a change in position of an individual during a medical imaging procedure for the individual. A reference image, in which the individual has a desired position for the medical imaging procedure, is obtained and processed to produce a reference contour bounding a region of interest. A later image (e.g. a current image) of the individual is obtained, and a display is controlled to provide a visual representation of the shape of the region of interest in the later image and, at least when one or more predetermined conditions are met, the reference contour.

Illustrative embodiments may, for example, be employed in medical imaging systems in which patient movement may affect a quality or clinical value of a medical image, for instance, in x-ray imaging systems.

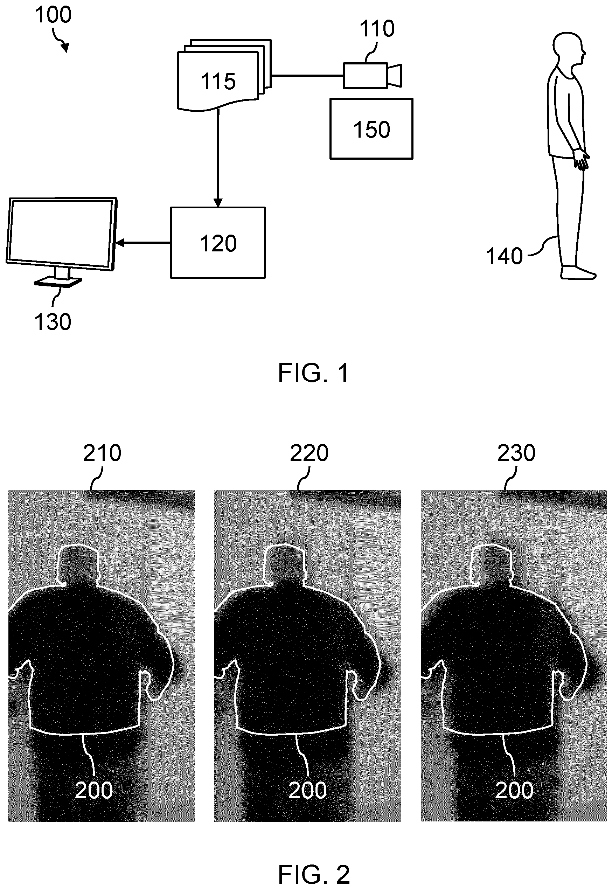

Fig. 1 illustrates an imaging system 100, according to an embodiment of the invention. The imaging system comprises a non-penetrative imaging device 110, a processing system 120 and a display device 130. The processing system is, itself, an embodiment of the invention.

The non-penetrative imaging device 110 is configured to capture images 115 of an individual 140 during a medical imaging procedure for the individual. In Fig. 1, the individual is undergoing a medical imaging procedure using medical imaging device 150.

The imaging system 100 may be used during any medical imaging procedure where movement of the individual being imaged may affect a quality or clinical value of medical images acquired during the medical imaging procedure. For instance, the medical imaging procedure may be an x-ray procedure.

The non-penetrative imaging device 110 may be any imaging device capable of acquiring an image from which a shape of the individual may be determined, using light of a non-penetrating wavelength (i.e. light that is reflected by the individual 140). For instance, the non-penetrative imaging device may be an RGB camera, an RGB-D camera or an infrared camera. Preferably, the non-penetrative imaging device is a device capable of acquiring depth information (i.e. a device comprising a depth sensor).

The images 115 captured by the non-penetrative imaging device 110 during a medical imaging procedure comprise at least a reference image of the individual 140 captured at a first point in time and a further image of the individual captured at a second point in time, as described in more detail below. The processing system 120 is configured to obtain at least the reference image and the further image from the non-penetrative imaging device.

The non-penetrative imaging device may acquire a sequence of images (e.g. a live image stream) during the medical imaging procedure, and the processing system may be configured to obtain each image captured by the non-penetrative imaging device immediately after the image is captured (i.e. the processing system may obtain and process each image in real time or close to real time).

The reference image of the individual captured at the first point in time is an image in which the individual 140 has a desired position for the medical imaging procedure, i.e. a position in which clinically useful medical images may be obtained. Preferably, the individual 140 is the only person included in the reference image, to ensure a reliability of the contour detection process described below.

In some examples, the reference image may be acquired based on user input from an imaging operator carrying out the medical imaging procedure or another clinical professional. For instance, the imaging operator may trigger acquisition of the reference image by the non-penetrative imaging device 110 in response to a determination by the imaging operator that a current position of the individual 140 is suitable for the medical imaging procedure (e.g. once the image operator has correctly positioned the individual or guided the individual into a desired position).

In some examples, an image acquired in response to the imaging operator triggering image acquisition may be analyzed to determine whether the imaging operator is present in the image. In response to a determination that the imaging operator is not present in the image (i.e. that the individual is the only person captured in the image), the image may be selected as the reference image. In response to a determination that the imaging operator is present in the image, a further image may be acquired and analyzed; this may be repeated until an image is acquired in which the imaging operator is not present. In other words, the reference image may be the first image captured by the non-penetrative imaging device, following image acquisition triggering, that does not contain the imaging operator.

In other examples, the processing system 120 may obtain a sequence of images captured by the non-penetrative imaging device 110, and automatically select, from the sequence of images, an image in which the individual 140 has a desired position as the reference image.

The processing system 120 may automatically select the reference image by processing each image in the sequence of images to determine whether the individual has a desired position for the medical imaging procedure in the image, for example, by using surface guided radiotherapy techniques. The use of surface guided radiotherapy to determine whether an individual is in a desired position for a medical imaging procedure is well known: see, for example, Freislederer et al. (2020), "Recent advanced in Surface Guided Radiation Therapy", Radiat Oncol., 15(1):187; and Al-Hallaq et al. (2022), "AAPM task group report 302: Surface-guided radiotherapy", Med Phys., 49(4):e82-e 1 12.

Alternatively, the processing system 120 may use a separate patient-position recognition system to determine a time at which the individual had a desired position for the medical imaging procedure, and select the image captured at the determined time as the reference image. Any known patient-position recognition system, such as a system employing lasers and skin markers or a separate surface guided radiotherapy system, may be used to determine the time at which the individual had a desired position for the medical imaging procedure.

Having obtained the reference image of the individual 140 from the non-penetrative imaging device 110, the processing system 120 performs contour detection on the reference image to produce a reference contour that bounds a representation of a region of interest of the individual within the reference image.

The region of interest is a region of the individual for which movement may affect a quality or clinical value of medical images acquired during the medical imaging procedure. The region of interest thus contains at least an intended anatomical target of the medical imaging procedure. In particular, the boundary of the region of interest may be defined by an outline of a portion of the individual in the image that includes the intended anatomical target.

The processing system 120 may use any suitable technique to perform contour detection on the reference image, examples of which will be readily apparent to the skilled person. For instance, where the reference image is a depth image, each pixel may be compared to a predetermined depth threshold: a pixel for which the distance from the detector does not exceed the predetermined depth threshold is classified as belonging to the individual while a pixel for which the distance from the detector exceeds the predetermined depth threshold is classified as not belonging to the individual. A pixel that belongs to the individual and has at least one immediately adjacent pixel not belonging to the individual may then be identified as a contour pixel.

Other techniques for performing contour detection on the reference image, including machine-learning techniques, will be readily apparent to the skilled person. For example, a change in intensity between the individual and the background may be used to detect an outline of the individual in the case of a grayscale or color reference image.

In some examples, the contour may be limited to a portion of the image coinciding with the field of view of the medical imaging device 150. In other words, pixels classified as belonging to the individual that correspond to a position of a border of the field of view of the medical imaging device 150 may be identified as contour pixels.

The further image of the individual 140 captured at the second point in time is an image taken at a later time than the reference image. The further image may be a current image of the individual at a time of visualizing a change in position of the individual (i.e. at a time of controlling the display device 130 to provide a visual representation, as described below).

Having obtained the further image of the individual 140 from the non-penetrative imaging device 110, the processing system 120 controls the display device 130 to provide a visualization of a change in position of the individual between the first point in time and the second point in time.

In particular, the processing system 120 controls the display device 130 to provide a visual representation of at least the shape of a representation of the region of interest within the further image (e.g. by displaying the further image itself and/or by displaying some other representation of the shape of the region of interest within the further image). The processing system further controls the display device to provide a visual representation of the reference contour, at least when movement of the region of interest between the first point in time and the second point in time meets one or more predetermined conditions.

In some examples, the non-penetrative imaging device 110 may capture a sequence of further images after the first point in time at which the reference image was taken, and the processing system 120 may repeat the steps of obtaining the further image and controlling the display device to provide a visualization of a change in position of the individual, compared with the first point in time, as each further image is captured. In other words, the display device 130 may provide a live visual representation of the individual's change in position between the first time, at which the reference image was captured, and a current time.

Various alternatives for visualizing a change in position of the individual are envisaged. Some example schemes for visualizing a change in position are provided in Figs. 2 to 9. Figs. 2 to 9 illustrate example visualizations of an individual who has been imaged during a chest x-ray procedure. The region of interest for which a reference contour has been produced (by performing contour detection on a reference image captured at a first time) is therefore an upper part of the individual's body (including the head, arms and upper torso). This reference contour bounds the outline of the part of the individual's body falling within the field of view of an x-ray device positioned to image the individual's chest.

Fig. 2 illustrates a first set of example visualizations that may be output at the display device 130 according to a first embodiment, in which a reference contour 200 bounding the position of the region of interest at the first point in time is superimposed on a further ("live") image of the individual. The reference contour is superimposed on the further image such that the position of the reference contour with respect to the further image indicates the position of the region of interest at the first time with respect to the position of the region of interest at the second time.

Example visualization 210 illustrates a change in the position of the individual between the first point in time (at which the reference image was captured) and a second point in time. Example visualization 210 shows no relevant movement of the individual has occurred between the first point in time and the second point in time, with the reference contour 200 indicating that the region of interest at the first point of time had the same position as the position of the region of interest in the further image captured at the second point in time. Irrelevant movement is movement of a region of the individual's body not containing an intended clinical target of the medical imaging procedure being carried out (e.g. movement of a region outside the region of interest and/or movement of a zone of the region of interest not containing the intended clinical target). In some examples, movement of the intended clinical target below a predetermined threshold may also be classified as irrelevant movement.

Example visualization 220 illustrates a change in the position of the individual between the first point in time and a third point in time. Example visualization 220 shows that a small amount of movement of the region of interest has occurred between the first point in time and the third point in time, with a slight misalignment between the reference contour 200 and the region of interest in the further image.

Example visualization 230 illustrates a change in the position of the individual between the first point in time and a fourth point in time. Example visualization 230 shows that a larger amount of movement of the region of interest has occurred between the first point in time and the fourth point in time, with a greater misalignment between the reference contour 200 and the region of interest in the further image.

In some examples, the display device 130 may provide a visual representation of the reference contour only when the movement of the region of interest between the first point in time and the later point in time meets the one or more predetermined conditions.

The one or more predetermined conditions may comprise a first condition that a measure of movement of the region of interest exceeds a first predetermined threshold. For instance, the first predetermined threshold may have a value suitable for distinguishing between relevant and irrelevant movement of the region of interest. As the skilled person will readily appreciate, a suitable value for the first predetermined threshold will depend on the particular use-case scenario (e.g. in the case of a chest x-ray, a first predetermined threshold in the range of 2-5 mm would enable irrelevant movement to be distinguished from relevant movement).

The measure of movement may, for instance, be a displacement of the region of interest between the first point in time and the later point in time.

The processing system 120 may determine whether the measure of movement of the region of interests exceeds the first predetermined threshold by performing contour detection on the further image to produce a further contour that bounds a representation of the region of interest within the further image. The processing system 120 may produce the further contour using any of the techniques described above in relation to the reference contour.

The processing system 120 may then process the reference contour and the further contour to determine the measure of movement. Techniques for determining a measure of movement, such as a displacement, between the reference contour and the further contour will be readily apparent to the skilled person. For instance, an iterative closest point algorithm may be used to determine a displacement between the reference contour and the further contour.

For example, in a first step, the distance between each pixel on the further contour and a corresponding closest pixel on the reference contour is calculated. In a second step, the calculated distances are processed to determine the displacement between the reference contour and the further contour (e.g. by calculating an average of a subset of the calculated distances that does not include the smallest and largest distance values).

Alternative techniques for determining a measure of movement of the region of interest (i.e. without requiring the further contour), will be apparent to the skilled person. For instance, where the images 115 captured by the non-penetrative imaging device are depth images, the depth information in the images may be used to detect movement of the surface of the individual (i.e. the measure of movement may be a measure of the surface change).

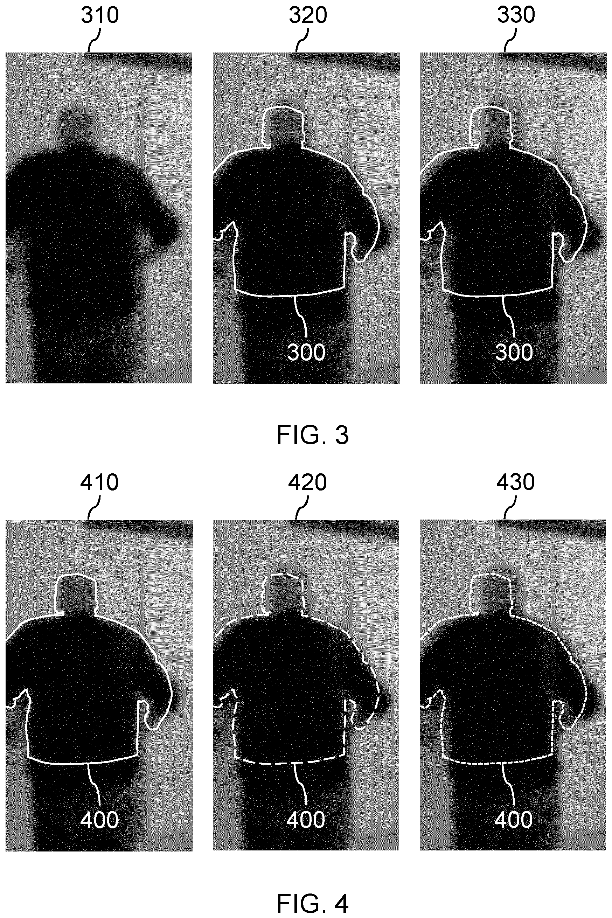

Fig. 3 illustrates a second set of example visualizations that may be output at the display device 130 according to a second embodiment, in which a reference contour 300 bounding the position of the region of interest at the first point in time is superimposed on a further ("live") image of the individual only in response to a determination that the measure of movement of the region of interest exceeds a first predetermined threshold.

Example visualization 310 illustrates a change in the position of the individual between the first point in time (at which the reference image was captured) and the second point in time represented by example visualization 210 in Fig. 2. As no relevant movement (i.e. movement of the intended clinical target, the individual's chest) has occurred between the first point in time and the second point in time, the measure of movement of the region of interest does not exceed the first predetermined threshold. Therefore, the reference contour is not included in the example visualization 310, and only the further image is shown.

Example visualization 320 illustrates a change in the position of the individual between the first point in time and the third point in time represented by example visualization 220 in Fig. 2. At the third point in time, the measure of movement of the region of interest exceeds the first predetermined threshold. Therefore, the reference contour 300 is superimposed on the further image in example visualization 320.

Example visualization 330 illustrates a change in the position of the individual between the first point in time and the fourth point in time represented by example visualization 230 in Fig. 2. At the fourth point in time, the measure of movement of the region of interest again exceeds the first predetermined threshold. Therefore, the reference contour 300 is superimposed on the further image in example visualization 330.

In some examples, one or more properties of the visual representation of the reference contour, when displayed, may change responsive to the measure of movement. For example, different colors may be used for the reference contour for different amounts of movement of the region of interest. For instance, a first color (e.g. green) may be used when the measure of movement does not exceed the first predetermined threshold, a second color (e.g. orange) may be used when the measure of movement exceeds the first predetermined threshold but does not exceed a second predetermined threshold, and a third color (e.g. red) may be used when the measure of movement exceeds the second predetermined threshold. Other properties of the visual representation of the reference contour, such as line type and thickness, that may be changed responsive to the measure of movement, will be readily apparent to the skilled person.

As the skilled person will appreciate, suitable values for the first predetermined threshold and second predetermined threshold will depend on a particular use-case scenario (e.g. dependent on examination type, and, in some cases, on hospital-specific guidelines). For instance, in the case of a chest x-ray, the first predetermined threshold may have a value in the range of 2-5 mm, and the second predetermined threshold may have a value in the range of 15-20 mm. In some examples, the first and/or second predetermined threshold may be defined by user input.

Fig. 4 illustrates a third set of example visualizations that may be output at the display device 130 according to a third embodiment, in which a line type of a reference contour 400 superimposed on the further image changes responsive to an amount of movement of the region of interest.

Example visualization 410 illustrates a change in the position of the individual between the first point in time (at which the reference image was captured) and the second point in time represented by example visualization 210 in Fig. 2. As no relevant movement has occurred between the first point in time and the second point in time, the measure of movement of the region of interest does not exceed the first predetermined threshold. The reference contour 400 in example visualization 410 is represented by a solid line.

Example visualization 420 illustrates a change in the position of the individual between the first point in time and the third point in time represented by example visualization 220 in Fig. 2. At the third point in time, the measure of movement of the region of interest exceeds the first predetermined threshold but does not exceed a second predetermined threshold. The reference contour 400 in example visualization 420 is represented by a dashed line.

Example visualization 430 illustrates a change in the position of the individual between the first point in time and the fourth point in time represented by example visualization 230 in Fig. 2. At the fourth point in time, the measure of movement of the region of interest exceeds the second predetermined threshold. The reference contour 400 in example visualization 430 is represented by a dotted line.

In some examples, the visual representation of at least the shape of the region of interest within the further image may comprise a visual representation of the further contour that bounds a representation of the region of interest within the further image. The further contour may be produced by performing contour detection on the further image, as described above.

The processing system 120 may control the display device 130 to provide a visual representation of the further contour at least when movement of the region of interest between the first point in time and the later point in time meets the one or more predetermined conditions.

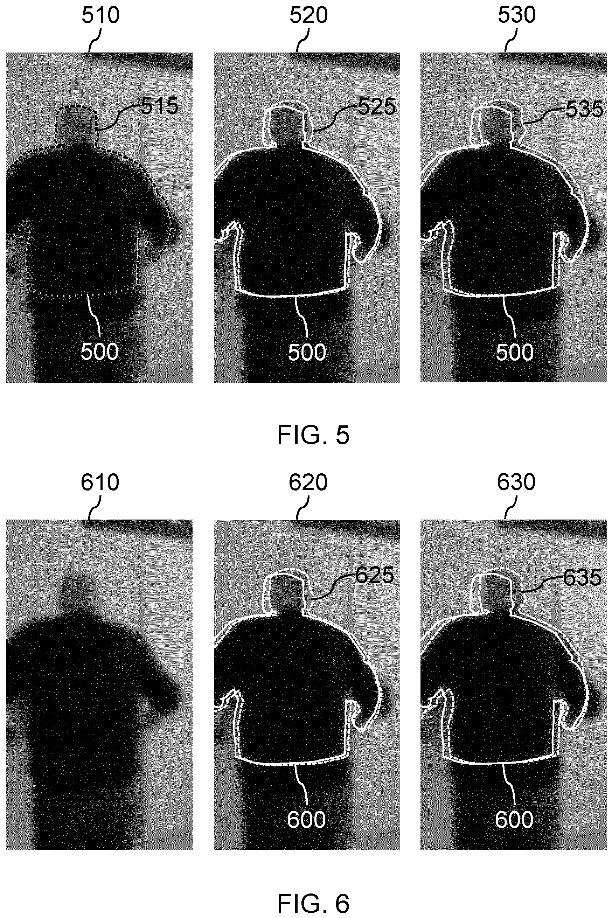

Fig. 5 illustrates a fourth set of example visualizations that may be output at the display device 130 according to a fourth embodiment, in which a reference contour 500, bounding the position of the region of interest at the first point in time, and a further contour, bounding the position of the region of interest in the further image, are both superimposed on the further image. In this embodiment, both the reference contour and the further contour are always superimposed on the further image (i.e. regardless of the amount of movement of the region of interest).

Example visualization 510 illustrates a change in the position of the individual between the first point in time (at which the reference image was captured) and the second point in time represented by example visualization 210 in Fig. 2. Reference contour 500, represented by a solid white line, and further contour 515, represented by a black dotted line, are superimposed on the further image captured at the third point in time. As no relevant movement has occurred between the first point in time and the second point in time, the reference contour 500 and the further contour 515 have the same position.

Example visualization 520 illustrates a change in the position of the individual between the first point in time and the third point in time represented by example visualization 220 in Fig. 2. Reference contour 500, represented by a solid line, and further contour 525, represented by a dotted line, are superimposed on the further image captured at the third point in time.

Example visualization 530 illustrates a change in the position of the individual between the first point in time and the fourth point in time represented by example visualization 230 in Fig. 2. Reference contour 500, represented by a solid line, and further contour 535, represented by a dotted line, are superimposed on the further image captured at the fourth point in time.

In some examples, the processing system 120 may control the display device 130 to provide a visual representation of the further contour only when movement of the region of interest between the first point in time and the later point in time meets the one or more predetermined conditions. For instance, the visual representation of the further contour may be provided only when a measure of movement of the region of interest (e.g. a displacement of the region of interest) exceeds the first predetermined threshold. The visual representation of the reference contour may be provided for all measures of movement, or may also be provided only in response to the measure of movement exceeding the first predetermined threshold (i.e. when the visual representation of the further contour is provided).

Fig. 6 illustrates a fifth set of example visualizations that may be output at the display device 130 according to a fifth embodiment, in which a reference contour 600 and a further contour are superimposed on a further ("live") image of the individual only in response to a determination that the measure of movement of the region of interest exceeds a first predetermined threshold.

Example visualization 610 illustrates a change in the position of the individual between the first point in time (at which the reference image was captured) and the second point in time represented by example visualization 210 in Fig. 2. As no relevant movement (i.e. movement of the intended clinical target, the individual's chest) has occurred between the first point in time and the second point in time, the measure of movement of the region of interest does not exceed the first predetermined threshold. Therefore, neither the reference contour nor the further contour are included in the example visualization 610, and only the further image is shown.

Example visualization 620 illustrates a change in the position of the individual between the first point in time and the third point in time represented by example visualization 220 in Fig. 2. At the third point in time, the measure of movement of the region of interest exceeds the first predetermined threshold. Therefore, the reference contour 600, represented by a solid line, and the further contour 625, represented by a dotted line, are superimposed on the further image in example visualization 620.

Example visualization 630 illustrates a change in the position of the individual between the first point in time and the fourth point in time represented by example visualization 230 in Fig. 2. At the fourth point in time, the measure of movement of the region of interest again exceeds the first predetermined threshold. Therefore, the reference contour 600, represented by a solid line, and the further contour 635, represented by a dotted line, are superimposed on the further image in example visualization 630.

In some examples, one or more properties of the visual representation of the further contour, when displayed, may change responsive to a measure of movement of the region of interest (e.g. a displacement of the region of interest). The measure of movement of the region of interest may be determined as described above.

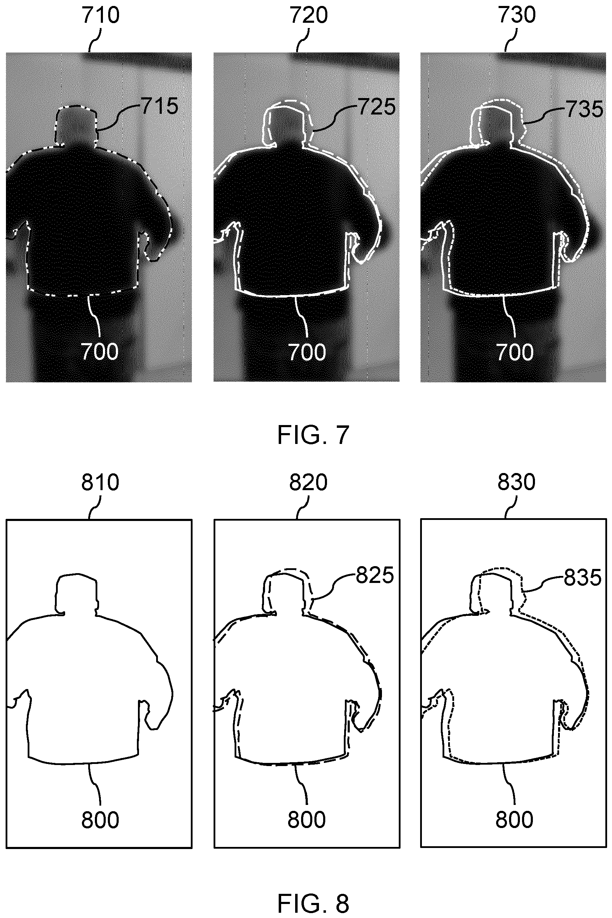

Fig. 7 illustrates a sixth set of example visualizations that may be output at the display device 130 according to a sixth embodiment, in which a visual representation of a further contour superimposed on the further image changes responsive to an amount of movement of the region of interest. A reference contour 700, bounding the position of the region of interest at the first point in time, is also superimposed on the further image, using a solid white line, in all example visualizations in Fig. 7.

Example visualization 710 illustrates a change in the position of the individual between the first point in time (at which the reference image was captured) and the second point in time represented by example visualization 210 in Fig. 2. As no relevant movement has occurred between the first point in time and the second point in time, the measure of movement of the region of interest does not exceed the first predetermined threshold. In example visualization 710, the further contour 715 is represented by a black dash-dotted line.

Example visualization 720 illustrates a change in the position of the individual between the first point in time and the third point in time represented by example visualization 220 in Fig. 2. At the third point in time, the measure of movement of the region of interest exceeds the first predetermined threshold but does not exceed a second predetermined threshold. The further contour 725 in example visualization 720 is represented by a dashed line.

Example visualization 730 illustrates a change in the position of the individual between the first point in time and the fourth point in time represented by example visualization 230 in Fig. 2. At the fourth point in time, the measure of movement of the region of interest exceeds the second predetermined threshold. The further contour 735 in example visualization 730 is represented by a dotted line.

Other changes to the visual representation of the further contour that may be made responsive to the measure of movement will be apparent to the skilled person. For instance, the color of the further contour may change responsive to the measure of movement of the region of interest, e.g. a first color may be used for the further contour in response to the measure of movement failing to exceed the first predetermined threshold, a second color may be used for the further contour in response to the measure of movement exceeding the first predetermined threshold but failing to exceed the second predetermined threshold, and a third color may be used for the further contour in response to the measure of movement exceeding the second threshold, while a fourth color may be used for the reference contour for all values of the measure of movement.

In some examples, the one or more properties of the further contour may change continuously responsive to the measure of movement, rather than changing discretely according to the value of the measure of movement relative to one or more thresholds. For instance, a shade or hue of the color of the further contour may depend on a value of the measure of movement, e.g. with a darkness of the shade increasing as the measure of movement increases.

In some examples, one or more properties of both the reference contour and the further contour may change responsive to the measure of movement. In some examples, the reference contour and/or the further contour may not be displayed when the measure of movement does not exceed the first predetermined threshold.

In some examples, the visualization of the change in position of the individual 140 may not include the further image. For instance, the processing system 120 may control the display device 130 to provide a visual representation only of the further contour, or a visual representation of the reference contour and the further contour. Such a visualization may be used, in particular, in situations in which the display device 130 is visible to people other than the individual being imaged and the imaging operator for the medical imaging procedure.

Fig. 8 illustrates a seventh set of example visualizations that may be output at the display device 130 according to a seventh embodiment, in which only contours are displayed. A reference contour 800, bounding the position of the region of interest at the first point in time is displayed using a solid line in all example visualizations in Fig. 8, with a position of the visual representation of the further contour with respect to the reference contour (or a lack of the further contour in the visualization) indicating a current position of the region of interest with respect to the position of the region of interest at the first point in time. The reference and further contours are displayed in a box, with the positions of each contour relative to the box indicating the position of the contour within the further image.

Example visualization 810 illustrates a change in the position of the individual between the first point in time (at which the reference image was captured) and the second point in time represented by example visualization 210 in Fig. 2. In example visualization 810, only the reference contour is displayed. As no relevant movement has occurred between the first point in time and the second point in time, the visual representation of the reference contour functions both as a visual representation of the reference contour and as a visual representation of the shape of the region of interest within the further image.

Example visualization 820 illustrates a change in the position of the individual between the first point in time and the third point in time represented by example visualization 220 in Fig. 2. At the third point in time, the measure of movement of the region of interest exceeds the first predetermined threshold but does not exceed a second predetermined threshold. The further contour 825 in example visualization 820 is represented by a dashed line.

Example visualization 830 illustrates a change in the position of the individual between the first point in time and the fourth point in time represented by example visualization 230 in Fig. 2. At the fourth point in time, the measure of movement of the region of interest exceeds the second predetermined threshold. The further contour 835 in example visualization 830 is represented by a dotted line.

In some examples, rather than omitting the further image from the visualization entirely, the processing system 120 may control the display device 130 to provide a visual representation of the further image that preserves the privacy of the individual 140. For instance, the display device may display a blurred or otherwise obscured version of the further image, or an abstraction of the further image (e.g. a silhouette of the individual). Where the non-penetrative imaging device 110 is capable of obtaining depth information, the display device may display a depth image.

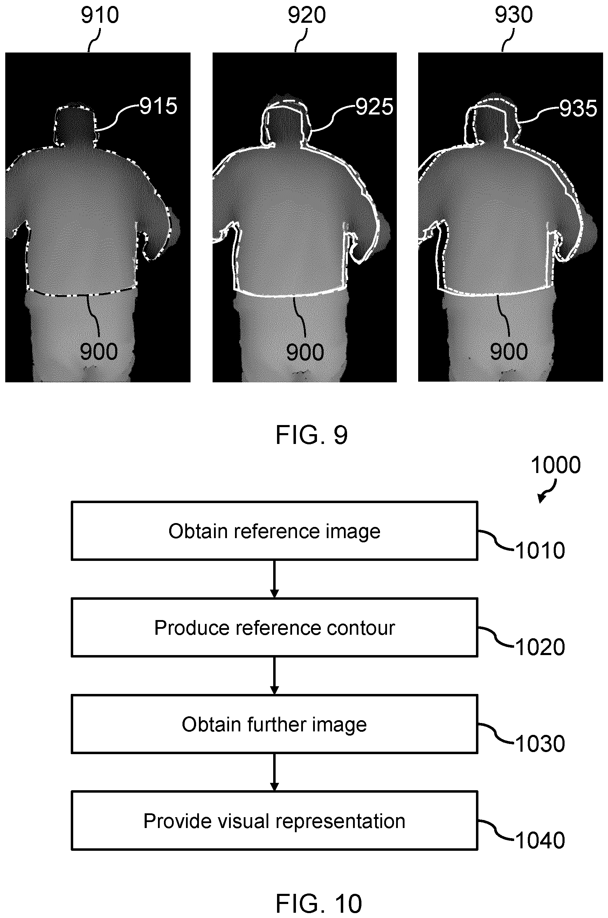

Fig. 9 illustrates an eighth set of example visualizations that may be output at the display device 130 according to an eighth embodiment, in which the reference contour 900 and a further contour are superimposed on a "live" depth image. The visual representation of the further contour changes responsive to an amount of movement of the region of interest, while the reference contour is represented by a solid white line in all example visualizations in Fig. 9.

Example visualization 910 illustrates a change in the position of the individual between the first point in time (at which the reference image was captured) and the second point in time represented by example visualization 210 in Fig. 2. The reference contour 900 and further contour 915 are superimposed on a depth image captured at the second point in time. As no relevant movement has occurred between the first point in time and the second point in time, the measure of movement of the region of interest does not exceed the first predetermined threshold. In example visualization 910, the further contour 915 is represented by a black dash-dotted line.

Example visualization 920 illustrates a change in the position of the individual between the first point in time and the third point in time represented by example visualization 220 in Fig. 2. The reference contour 900 and further contour 925 are superimposed on a depth image captured at the third point in time. At the third point in time, the measure of movement of the region of interest exceeds the first predetermined threshold but does not exceed a second predetermined threshold. The further contour 925 in example visualization 920 is represented by a dashed line.

Example visualization 930 illustrates a change in the position of the individual between the first point in time and the fourth point in time represented by example visualization 230 in Fig. 2. The reference contour 900 and further contour 935 are superimposed on a depth image captured at the fourth point in time. At the fourth point in time, the measure of movement of the region of interest exceeds the second predetermined threshold. The further contour 935 in example visualization 930 is represented by a dotted line.

The further images of the individual included in the example visualizations of the Figs. are blurred grayscale images. However, the skilled person will readily appreciate that the visualization provided at the display device 130 may instead include an image that is colored (e.g. a RGB image) and/or that has a high resolution. Similarly, while the contours superimposed on the images in the example visualizations are black or white, colored contours may also be used. In some examples, the reference contour and/or the further contour may be represented by a colored line (i.e. not black, white or grey) superimposed on a greyscale further image.

Where more than one color is used in the visualization provided at the display device 130, the colors may be selected such that people with color blindness are able to distinguish between the displayed colors.

In some examples, the visualization may include additional elements. For instance, the visualization may further comprise a textual overlay providing a value for the measure of movement of the region of interest. In some examples, the value of the measure of movement may be displayed only when the measure of movement exceeds the first predetermined threshold, or only when the measure of movement exceeds the second predetermined threshold.

Fig. 10 illustrates a computer-implemented method 1000 for visualizing a change in position of an individual during a medical imaging procedure for the individual, according to an embodiment of the invention.

The method 1000 begins at step 1010, at which a reference image of an individual is obtained. The reference image is an image that was captured at a first point in time using a non-penetrative imaging device. In some examples, the reference image may be a depth image.

At step 1020, contour detection is performed on the reference image to produce a reference contour that bounds a representation of a region of interest of the individual within the reference image. In some examples, step 1020 comprises performing threshold-based contour detection on the reference image.

At step 1030, a further image of the individual is obtained. The further image is an image that was captured at a second point in time using the non-penetrative imaging device.

At step 1040, a display is controlled to provide a visual representation of at least the shape of a representation of the region of interest within the further image, and, at least when movement of the region of interest between the first point in time and the second point in time meets one or more predetermined conditions, the reference contour.

In some examples, the step 1040 of controlling the display comprises controlling the display to provide a visual representation of the reference contour only when the movement of the region of interest between the first point in time and the second point in time meets the one or more predetermined conditions.

The one or more predetermined conditions may comprise a first condition that a measure of movement exceeds a first predetermined threshold. In some examples, the measure of movement may be determined by performing contour detection on the further image to produce a further contour, as described above, and processing the reference contour and the further contour to determine the measure of movement. The measure of movement may be a displacement of the region of interest between the first point in time and the second point in time.

In some examples, wherein one or more properties of the visual representation of the reference contour, when displayed, may change responsive to the measure of movement.

In some examples, the computer-implemented method 1000 may further comprise a step (not shown in Fig. 10) of performing contour detection on the further image to produce a further contour that bounds a representation of the region of interest within the further image; and the step 1040 of controlling the display to provide the visual representation of at least the shape of the representation of the region of interest within the further image may comprise, at least when movement of the region of interest between the first point in time and the second point in time meets the one or more predetermined conditions, controlling the display to provide a visual representation of the further contour.

In some examples, the step 1040 of controlling the display (130) may comprise controlling the display to provide a visual representation of the further contour only when the movement of the region of interest between the first point in time and the second point in time meets the one or more predetermined conditions.

In some examples, one or more properties of the visual representation of the further contour, when displayed, may change responsive to the measure of movement.

In some examples, the step 1040 of controlling the display to provide the visual representation of at least the shape of the representation of the region of interest within the further image may consist of controlling the display to provide a visual representation of the further contour.

In other examples, the step 1040 of controlling the display to provide the visual representation of at least the shape of the representation of the region of interest within the further image may comprise controlling the display to provide a visual representation of the further image. The visual representation of the further image may be the further image or an abstraction of the further image.

In some examples, steps 1030 and 1040 may be repeated, obtaining a new further image at each later point in time (e.g. a third point in time, a fourth point in time, etc.), and controlling the display to visualize a change in position of the individual between the first point in time and the later point in time (e.g. a current time). For instance, the non-penetrative imaging device may continuously capture images of the individual, and the display may be controlled to provide a visual representation of at least the shape of a representation of the region of interest within the further image each time a new further image is acquired, such that the display provides a "live" visual representation of the region of interest.

In some examples, the entire method may be repeated, obtaining a new reference image at a fifth point in time and providing a visualization of a change in position of the individual with respect to the fifth point in time. A new reference image may, for example, be obtained in response to a change in the system geometry of the medical imaging system used to acquire medical imaging data for the individual (e.g. tube or detector movement in the case of an x-ray imaging system). The new reference image may be acquired in response to the imaging operator triggering capture of a new reference image, as described above, or automatically, in response to a determination that the system geometry has changed since acquisition of the previous reference image.

It will be understood that the disclosed methods are computer-implemented methods. As such, there is also proposed a concept of a computer program comprising code means for implementing any described method when said program is run on a processing system. There is also proposed a computer-readable medium comprising instructions which, when executed by a computer, cause the computer to carry out the steps of any described method.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method (1000) for visualizing a change in position of an individual (140) during a medical imaging procedure for the individual, the computer-implemented method comprising:
obtaining a reference image of an individual captured at a first point in time using a non-penetrative imaging device (110);
performing contour detection on the reference image to produce a reference contour (200, 300, 400, 500, 600, 700, 800, 900) that bounds a representation of a region of interest of the individual within the reference image;
obtaining a further image (210, 220, 230, 310, 320, 330, 410, 420, 430, 510, 520, 530, 610, 620, 630, 710, 720, 730, 810, 820, 830, 910, 920, 930) of the individual captured at a second point in time using the non-penetrative imaging device; and
controlling a display (130) to provide a visual representation of:
at least the shape of a representation of the region of interest within the further image; and
at least when movement of the region of interest between the first point in time and the second point in time meets one or more predetermined conditions, the reference contour.

2. The computer-implemented method (1000) of claim 1, wherein the step of controlling the display (130) comprises controlling the display to provide a visual representation of the reference contour (200, 300, 400, 500, 600, 700, 800, 900) only when the movement of the region of interest between the first point in time and the second point in time meets the one or more predetermined conditions.

3. The computer-implemented method (1000) of claim 1 or 2, wherein the one or more predetermined conditions comprises a first condition that a measure of movement exceeds a first predetermined threshold.

4. The computer-implemented method (1000) of claim 3, further comprising:
performing contour detection on the further image (210, 220, 230, 310, 320, 330, 410, 420, 430, 510, 520, 530, 610, 620, 630, 710, 720, 730, 810, 820, 830, 910, 920, 930) to produce a further contour (515, 525, 535, 625, 635, 715, 725, 735, 825, 835, 915, 925, 935) that bounds a representation of the region of interest within the further image; and
processing the reference contour (200, 300, 400, 500, 600, 700, 800, 900) and the further contour to determine the measure of movement.

5. The computer-implemented method (1000) of claim 3 or 4, wherein the measure of movement is a displacement of the region of interest between the first point in time and the second point in time.

6. The computer-implemented method (1000) of any of claim 3 to 5, wherein one or more properties of the visual representation of the reference contour (200, 300, 400, 500, 600, 700, 800, 900), when displayed, changes responsive to the measure of movement.

7. The computer-implemented method (1000) of any of claims 1 to 6, wherein:
the computer-implemented method further comprises performing contour detection on the further image (210, 220, 230, 310, 320, 330, 410, 420, 430, 510, 520, 530, 610, 620, 630, 710, 720, 730, 810, 820, 830, 910, 920, 930) to produce a further contour (515, 525, 535, 625, 635, 715, 725, 735, 825, 835, 915, 925, 935) that bounds a representation of the region of interest within the further image; and
the step of controlling the display (130) to provide the visual representation of at least the shape of the representation of the region of interest within the further image comprises, at least when movement of the region of interest between the first point in time and the second point in time meets the one or more predetermined conditions, controlling the display to provide a visual representation of the further contour.

8. The computer-implemented method (1000) of claim 7, wherein the step of controlling the display (130) comprises controlling the display to provide a visual representation of the further contour (515, 525, 535, 625, 635, 715, 725, 735, 825, 835, 915, 925, 935) only when the movement of the region of interest between the first point in time and the second point in time meets the one or more predetermined conditions.

9. The computer-implemented method (1000) of claim 7 or 8, when dependent on at least claim 3, wherein one or more properties of the visual representation of the further contour (515, 525, 535, 625, 635, 715, 725, 735, 825, 835, 915, 925, 935), when displayed, changes responsive to the measure of movement.

10. The computer-implemented method (1000) of any of claims 7 to 9, wherein the step of controlling the display (130) to provide the visual representation of at least the shape of the representation of the region of interest within the further image (210, 220, 230, 310, 320, 330, 410, 420, 430, 510, 520, 530, 610, 620, 630, 710, 720, 730, 810, 820, 830, 910, 920, 930) consists of controlling the display to provide a visual representation of the further contour (515, 525, 535, 625, 635, 715, 725, 735, 825, 835, 915, 925, 935).

11. The computer-implemented method (1000) of any of claims 1 to 9, wherein the step of controlling the display (130) to provide the visual representation of at least the shape of the representation of the region of interest within the further image (210, 220, 230, 310, 320, 330, 410, 420, 430, 510, 520, 530, 610, 620, 630, 710, 720, 730, 810, 820, 830, 910, 920, 930) comprises controlling the display to provide a visual representation of the further image.

12. The computer-implement method (1000) of claim 11, wherein the visual representation of the further image is an abstraction of the further image.

13. The computer-implemented method (1000) of 1 to 12, wherein the reference image is a depth image.

14. The computer-implemented method (1000) of any of claims 1 to 13, wherein the step of performing contour detection comprises performing threshold-based contour detection.

15. A processing system (120) for visualizing a change in position of an individual (140) during a medical imaging procedure for the individual, the processing system being configured to:
obtain a reference image of an individual captured at a first point in time using a non-penetrative imaging device (110);
perform contour detection on the reference image to produce a reference contour (200, 300, 400, 500, 600, 700, 800, 900) that bounds a representation of a region of interest of the individual within the reference image;
obtain a further image (210, 220, 230, 310, 320, 330, 410, 420, 430, 510, 520, 530, 610, 620, 630, 710, 720, 730, 810, 820, 830, 910, 920, 930) of the individual captured at a second point in time using the non-penetrative imaging device; and
control a display (130) to provide a visual representation of:
at least the shape of a representation of the region of interest within the further image; and
at least when movement of the region of interest between the first point in time and the second point in time meets one or more predetermined conditions, the reference contour.
